# EUROPEAN PATENT APPLICATION

(11) **EP 4 699 586 A1**
(43) Date of publication of application: **25.02.2026**
(21) Application number: 25196290.8
(22) Date of filing: 15.08.2025
(51) Int. Cl.: A61F 13/49, A61F 13/496, A61F 13/505

(54) **WASHABLE INCONTINENCE UNDERGARMENT**

(30) Priority: 19.08.2024 US 202463684462 P
(71) Applicant: Sleaks IP Corporation, Mississauga ON L5C 3V5 (CA)
(72) Inventor: Vurinaris, Katheryn Diane, Mississauga, ON L5C 3V5 (CA)
(74) Representative: FRKelly

(57) **Abstract**

An undergarment including a washable absorbent insert and a main body is disclosed. The main body includes a front portion, a rear portion, and a crotch portion having a pelvic end that adjoins the front portion and a buttock end that adjoins the rear portion. The crotch portion has a pocket for retaining the insert therein. The pocket is defined by a pocket outer wall and a pocket inner wall. The pocket outer wall is absorbent and configured to retain liquid therein the pocket. The pocket inner wall is at least water-resistant and proximal to a wearer when the undergarment is worn. The pocket inner wall has a face defining a longitudinal opening. The longitudinal opening is sized to allow for insertion and removal of the insert from the pocket. The longitudinal opening allows for liquid to be deposited directly on a wicking layer therein the pocket.

## Description

### FIELD

The application relates to the field of undergarments, and more specifically to the field of incontinence undergarments.

### INTRODUCTION

Many suffer from various bladder related conditions, such as but not limited to urinary stress incontinence, over-active bladder issues, including but not limited to postpartum, bladder prolapse, urinary tract infections, prostate issues, and other age-related bladder weakens or injuries.

Standard underwear offers very little to no absorbency and thus provides little to no protection for those who suffer from incontinence. Existing incontinence products are typically single-use. However, single-use products must be discarded, which can generate significant waste. Single-use products can also be expensive over time, or for institutional facilities with a large number of individuals (e.g., hospitals or long-term care homes). In addition, individuals can be reluctant to adopt disposable undergarments for various reasons, such as discomfort or embarrassment.

### SUMMARY

The following summary is intended to introduce the reader to various aspects of the applicant's teaching, but not to define any invention.

In a first aspect, in at least one embodiment, an undergarment including a washable absorbent insert and a main body is disclosed. The main body includes a front portion, a rear portion opposite to the front portion, and a crotch portion having a pelvic end and a buttock end. The pelvic end of the crotch portion adjoins a bottom edge of the front portion. The buttock end of the crotch portion adjoins a bottom edge of the rear portion. The crotch portion has a pocket for retaining the insert therein. The pocket is defined by a pocket outer wall and a pocket inner wall. The pocket outer wall is absorbent and configured to retain liquid therein the pocket. The pocket inner wall is at least water-resistant and proximal to a wearer when the undergarment is worn. The pocket inner wall has a face defining a longitudinal opening. The longitudinal opening is sized to allow for insertion and removal of the insert from the pocket. The longitudinal opening allows for liquid to be deposited directly on a wicking layer therein the pocket.

In various embodiments, the pocket outer wall forms a liquid collecting area.

In various embodiments, the pocket comprises a perimeter; and the pocket is enclosed along the perimeter.

In various embodiments, the pocket inner wall is bonded to the pocket outer wall along the perimeter of the pocket.

In various embodiments, the pocket inner wall comprises a plurality of water-resistant layers bonded together.

In various embodiments, the plurality of water-resistant layers extend across the entirety of the pocket inner wall between the perimeter and the longitudinal opening.

In various embodiments, the plurality of water-resistant layers comprise polyurethane.

In various embodiments, each layer of the plurality of water-resistant layers is at least water-repellent.

In various embodiments, each layer of the plurality of water-resistant layers is water-proof.

In various embodiments, the crotch portion is seamless.

In various embodiments, the insert comprises a plurality of insert absorbent layers sandwiched between the two insert wicking layers, the insert wicking layers being configured to draw liquids toward the plurality of insert absorbent layers.

In various embodiments, each of the insert wicking layers comprise a knitted twill fabric.

In various embodiments, the knitted twill fabric comprises polyester and spandex.

In various embodiments, each of the plurality of insert absorbent layers comprise a microfiber loop terry fabric.

In various embodiments, the microfiber loop terry fabric comprise polyester and nylon.

In various embodiments, the insert is reversible.

In various embodiments, the pocket outer wall comprises at least a pocket wicking layer, a pocket lining layer, and at least one pocket absorbent layer between the pocket wicking layer and the pocket lining layer, the pocket wicking layer configured to draw liquids toward to the at least one pocket absorbent layer, the pocket lining layer being at least water-resistant and configured to reduce liquid egress from the pocket outer wall.

In various embodiments, the pocket wicking layer comprises a knitted twill fabric.

In various embodiments, the knitted twill fabric comprises polyester and spandex.

In various embodiments, each of the at least one pocket absorbent layers comprise a microfiber loop terry fabric.

In various embodiments, the microfiber loop terry fabric comprise polyester and nylon.

In various embodiments, the pocket lining layer comprises a plurality of water-resistant layers bonded together.

In various embodiments, the plurality of water-resistant layers comprise polyurethane.

In various embodiments, the pocket lining layer is at least water-repellent.

In various embodiments, the pocket lining layer is water-proof.

In various embodiments, an upper edge of the main body comprises an elastic waist band.

In various embodiments, the front portion and the rear portion comprise a breathable fabric.

In various embodiments, the front portion and the rear portion comprise a biodegradable fabric.

In various embodiments, the front portion and the rear portion comprise an organic bamboo fabric.

In various embodiments, the main body forms a pair of briefs.

In various embodiments, the briefs are bikini briefs.

In various embodiments, the undergarment, including the insert, can be washable, allowing it to be less expensive over time. Moreover, by being washable, the undergarment creates less waste, which is better environmentally. In addition, the washable undergarment can be worn more discretely or inconspicuously.

### BRIEF DESCRIPTION OF THE DRAWINGS

For a better understanding of the described examples and to show more clearly how they may be carried into effect, reference will now be made, by way of example, to the accompanying drawings in which:
FIG. 1A is a front view of a main body and an insert of an example undergarment, in accordance with some embodiments;
FIG. 1B is a rear view of the main body of the example undergarment of FIG. 1A;
FIG. 2A is a front view of the interior of the main body of the example undergarment of FIG. 1A;
FIG. 2B is a rear view of the interior of the main body of the example undergarment of FIG. 1A;
FIG. 2C is a magnified, cross-sectional view of the pocket of the example undergarment of FIG. 1A;
FIG. 3A is a front view of the interior of a main body and an insert of another example undergarment, in accordance with some embodiments;
FIG. 3B is a view of the insert of FIG. 3A being inserted in the main body of the undergarment of FIG. 3A;
FIG. 3C is a view of a disposable insert positioned within the pocket of the undergarment of FIG. 3A;
FIG. 4 is a schematic view of multiple fabric layers of the example undergarment of FIG. 1A;
FIG. 5A is an illustration of a water-proof fabric, in accordance with some embodiments;
FIG. 5B is an illustration of an example knitted twill fabric, in accordance with some embodiments; and
FIG. 5C is an illustration of an example microfiber loop terry fabric, in accordance with some embodiments.

### DESCRIPTION OF VARIOUS EMBODIMENTS

Numerous embodiments are described in this application, and are presented for illustrative purposes only. The described embodiments are not intended to be limiting in any sense. The invention is widely applicable to numerous embodiments, as is readily apparent from the disclosure herein. Those skilled in the art will recognize that the present invention may be practiced with modification and alteration without departing from the teachings disclosed herein. Although particular features of the present invention may be described with reference to one or more particular embodiments or figures, it should be understood that such features are not limited to usage in the one or more particular embodiments or figures with reference to which they are described.

The terms "an embodiment," "embodiment," "embodiments," "the embodiment," "the embodiments," "one or more embodiments," "some embodiments," and "one embodiment" mean "one or more (but not all) embodiments of the present invention(s)," unless expressly specified otherwise.

The terms "including," "comprising" and variations thereof mean "including but not limited to," unless expressly specified otherwise. A listing of items does not imply that any or all of the items are mutually exclusive, unless expressly specified otherwise. The terms "a," "an" and "the" mean "one or more," unless expressly specified otherwise.

As used herein and in the claims, two or more parts are said to be "coupled", "connected", "attached", "joined", "affixed", or "fastened" where the parts are joined or operate together either directly or indirectly (i.e., through one or more intermediate parts), so long as a link occurs. As used herein and in the claims, two or more parts are said to be "directly coupled", "directly connected", "directly attached", "directly joined", "directly affixed", or "directly fastened" where the parts are connected in physical contact with each other. As used herein, two or more parts are said to be "rigidly coupled", "rigidly connected", "rigidly attached", "rigidly joined", "rigidly affixed", or "rigidly fastened" where the parts are coupled so as to move as one while maintaining a constant orientation relative to each other. None of the terms "coupled", "connected", "attached", "joined", "affixed", and "fastened" distinguish the manner in which two or more parts are joined together.

Further, although method steps may be described (in the disclosure and / or in the claims) in a sequential order, such methods may be configured to work in alternate orders. In other words, any sequence or order of steps that may be described does not necessarily indicate a requirement that the steps be performed in that order. The steps of methods described herein may be performed in any order that is practical. Further, some steps may be performed simultaneously.

As used herein and in the claims, a group of elements are said to 'collectively' perform an act where that act is performed by any one of the elements in the group, or performed cooperatively by two or more (or all) elements in the group.

Some elements herein may be identified by a part number, which is composed of a base number followed by an alphabetical or subscript-numerical suffix (e.g. 112a, or 112₁). Multiple elements herein may be identified by part numbers that share a base number in common and that differ by their suffixes (e.g. 112₁, 112₂, and 112₃). All elements with a common base number may be referred to collectively or generically using the base number without a suffix (e.g. 112).

Some incontinence underwear are single-use, or disposable so that it is unnecessary to clean them between uses. However, disposable underwear can be undesirable from an environmental perspective. Disposable underwear can also be expensive, particularly if used in an institutional setting with multiple users.

According to some aspects of the present disclosure, the undergarment can be washable, which allows it to be less expensive over time. In addition, by being washable, the undergarment creates less waste, which is better environmentally. In addition, the washable undergarment can be worn more discretely or inconspicuously.

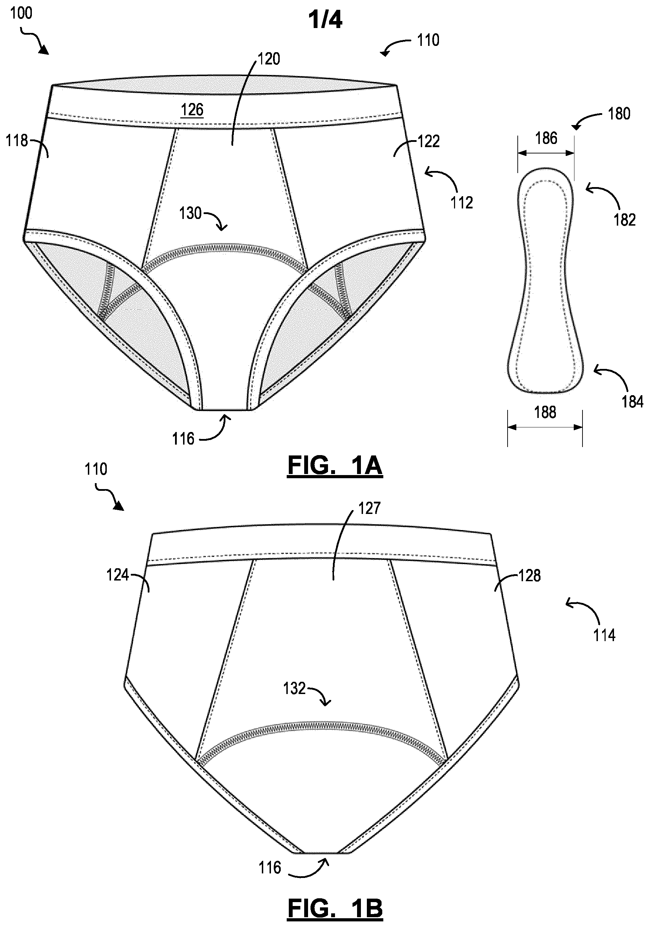

Reference is now made to FIG. 1A, which shows a front view of an example undergarment 100, in accordance with some embodiments. The undergarment 100 is a pair of underwear. The undergarment 100 is a pair of women's underwear. The undergarment 100 is a pair of briefs. In some embodiments, the undergarment 100 can be other types of briefs such as but not limited to bikini briefs, hipster briefs, boyshort briefs, high-rise briefs, French cut briefs, or mid-rise briefs.

The undergarment 100 includes a main body 110 and an insert 180 that can be placed within the main body 110. The main body 110 of the undergarment 100 has a front portion 112, a rear portion 114, a crotch portion 116, and a waist band 126. The rear portion 114 is opposite to the front portion 112. As shown in FIG. 1A, the front portion 112 can include a front left portion 118, a front center portion 120, and a front right portion 122.

Reference is now made to FIG. 1B, which shows a rear view of the undergarment 100. The rear portion 114 can include a rear left portion 124, a rear center portion 127, and a rear right portion 128. Although each of the front portion 112 and the rear portion 114 are shown with three portions or subportions, in some embodiments, the front portion 112 and the rear portion 114 can include fewer or more subportions. For example, the rear portion 114 can be a unitary piece. In another example, the front portion 112 can include five portions. In yet another example, the front left portion 118 and the rear right portion 128 can be a unitary piece. In yet another example, the front right portion 122 and the rear left portion 124 can be a unitary piece. In some embodiments, the number of subportions included in the front portion 112 and the rear portion 114 can affect the dimensional stability of the undergarment 100. For example, the undergarment 100 of FIG. 1A can maintain dimensional stability through at least 100 washes.

The waist band 126 forms an upper edge of the main body 110. In particular, the upper edge of the front portion 112 and the upper edge of the rear portion 114 adjoin the waist band 126. In some embodiments, the front portion 112 and the rear portion 114 can be stitched to the waist band 126. The waist band 126 supports the weight of the undergarment 100, especially when the undergarment 100 is soiled with liquids. In some embodiments, the waist band 126 is elastic.

In some embodiments, the front portion 112, the rear portion 114, or both the front portion 112 and the rear portion 114 can be formed of a breathable fabric, which can allow for more comfort to the wearer. In some embodiments, the front portion 112, the rear portion 114, or both the front portion 112 and the rear portion 114 can be a biodegradable fabric, which can be more desirable from an environmental perspective. In some embodiments, the front portion 112, the rear portion 114, or both the front portion 112 and the rear portion 114 can be both a breathable and a biodegradable fabric, such as but not limited to organic bamboo.

The crotch portion 116 extends from a pelvic end 130 to a buttock end 132. The pelvic end 130 of the crotch portion 116 adjoins a bottom edge of the front portion 112. The buttock end 132 of the crotch portion 116 adjoins a bottom edge of the rear portion 114. As shown, each of the pelvic end 130 and the buttock end 132 can be convex, that is, round out towards the front portion 112 and the rear portion 114.

The insert 180 is an absorbent insert. As shown in FIG. 1A, the insert 180 has a longitudinal axis that extends from an insert pelvic end 182 to an insert buttock end 184. As shown, the insert pelvic end 182 can have a width 186 that is narrower than the width 188 of the insert buttock end 184. As shown, the insert pelvic end 182 and the insert buttock end 184 can be convex, that is, round outwards.

The insert 180 can include multiple layers. In some embodiments, the absorbent capacity of the insert 180 can depend on the number of layers. In some other embodiments, the absorbent capacity of the insert 180 can depend on the thickness of one or more layers. In some embodiments, the insert 180 can absorb up to at least 150 milliliters. In some embodiments, the insert 180 can absorb up to at least 125 milliliters.

In some embodiments, the insert 180 is a washable insert. In some other embodiments, the insert 180 is a disposable insert. In further embodiments, the insert 180 is a limited-use insert that can be washed a predetermined number of times before being discarded.

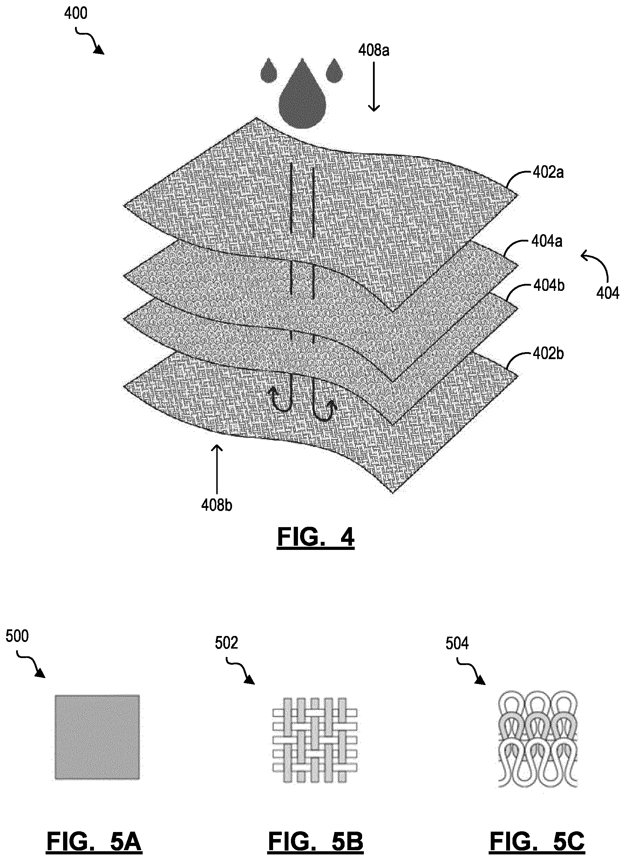

Reference now is made to FIG. 4, which shows an example of multiple layers 400, which can be used in the insert 180. For example, the insert 180 can include a plurality of insert absorbent layers 404a, 404b (collectively referred to as the plurality of insert absorbent layers 404) between two insert wicking layers 402a, 402b. With insert wicking layers 402a, 402b on either side of the insert 180, at least one of the insert wicking layers 402a, 402b are proximal to the wearer when positioned in the undergarment 100 and the undergarment is worn 100. That is, the insert 180 is reversible and can be used on either side.

The insert wicking layers 402a, 402b can be formed of any appropriate fabric for drawing liquids in one direction, more specifically, in a direction 408a, 408b toward the plurality of insert absorbent layers 404. In some embodiments, the insert wicking layer 402a, 402b can be a knitted twill fabric. FIG. 5B shows an example illustration of a knitted twill fabric 502. In some embodiments, the insert wicking layer 402a, 402b can be formed of polyester and spandex. For example, the insert wicking layer 402a, 402b can be 97% polyester and 3% spandex. Other fabric contents are possible. In some embodiments, the insert wicking layer 402a, 402b can be a polyester and spandex knitted twill fabric.

In some embodiments, the plurality of insert absorbent layers 404 can include two layers 404a, 404b. In other embodiments, the plurality of insert absorbent layers 404 can include more than two layers. For example, in one embodiment, the plurality of insert absorbent layers 404 includes three absorbent layers. With the plurality of insert absorbent layers 404 stacked together, liquid can be held between the plurality of layers within the stack. As a result, the stack can absorb more liquid than that of the sum of separate, individual layers alone.

The plurality of insert absorbent layers 404 can be formed of any appropriate fabric for absorbing liquids. In some embodiments, the plurality of insert absorbent layers 404 can be a microfiber loop terry fabric. FIG. 5C shows an example illustration of a microfiber loop terry fabric 504. In some embodiments, the plurality of insert absorbent layers 404 can be formed of polyester and nylon. For example, the plurality of insert absorbent layers 404 can be 88% polyester and 12% nylon. Other fabric contents are possible. In some embodiments, the insert absorbent layer can be a polyester and nylon microfiber loop terry fabric. In some embodiments, the plurality of insert absorbent layers 404 includes two layers 404a, 404b, but the layers are formed of heavier fabric to absorb more liquid.

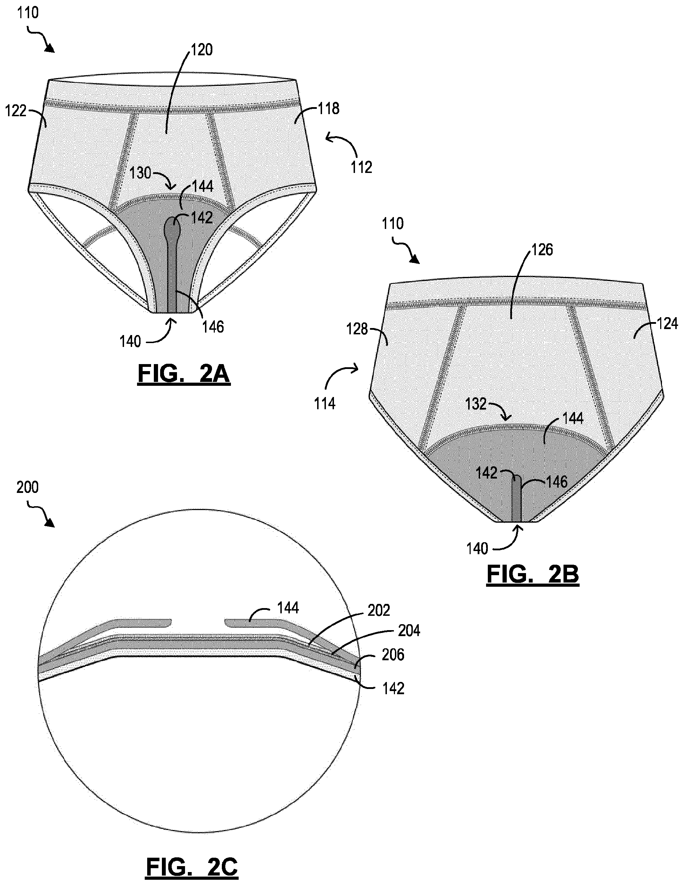

Reference is now made to FIG. 2A and FIG. 2B, which shows a front view and a rear view of the interior of the main body 110. The crotch portion 116 includes a pocket 140 for retaining an insert therein. The insert can be any absorbent insert, such as washable, absorbent insert 180. In some other cases, the insert can be a disposable, absorbent insert. Further, the main body 110, 410 can be used with any insert 180.

The pocket 140 is defined by a pocket outer wall 142 and a pocket inner wall 144. The pocket inner wall 144 is adjacent to the pocket outer wall 142. As shown in FIG. 2B, the pocket inner wall 144 is proximal to a wearer when the undergarment 100 is worn. In various embodiments, the pocket inner wall 144 can retain an insert 180 in position without additional attachment means, such as but not limited to adhesives, buttons, clips, or other attachment means. In some other embodiments, the pocket inner wall 144 retains an insert 180 in position using attachment means, such as but not limited to adhesives, buttons, clips, or other attachment means.

The pocket 140 has a perimeter. The pocket 140 is enclosed along the perimeter. In some embodiments, the pocket inner wall 144 can be bonded to the pocket outer wall 142 along the perimeter of the pocket 140, as shown in the magnified cross-sectional view of the pocket 140 in FIG. 2C.

The pocket inner wall 144 has a face defining a longitudinal opening 146. The longitudinal opening 146 is sized to allow for insertion and removal of an insert from the pocket 140. The longitudinal opening 146 allows for liquid to be deposited directly into the pocket 140. For example, when the insert 180 is positioned in the pocket 140, liquid, such as urine or other bodily fluids, can be deposited directly on the insert 180. More specifically, the liquid can be deposited directly on the insert wicking layer 402a, 402b of the insert 180 when the insert 180 is positioned in the pocket 140. That is, the insert wicking layer 402a, 402b is proximal to the pocket inner wall 144 when the insert is positioned in the undergarment 100. When the pocket 140 is empty, that is no insert is in the pocket 140, liquids can be deposited directly on the pocket outer wall 142.

The pocket inner wall 144 can be seamless, which can be more comfortable for wearers as the pocket inner wall 144 is in contact with the wearer. The pocket inner wall 144 can be formed of a plurality of water-resistant layers bonded together. The plurality of water-resistant layers of the pocket inner wall 144 can extend across the entirety of the pocket inner wall 144, between the perimeter of the pocket and the central, longitudinal opening 146.

The pocket inner wall 144 can be formed of any appropriate fabric to reduce liquid egress from pocket inner wall 144, that is, out of the pocket 140. Liquid egress out of the pocket inner wall 144 would result in wetness in contact with the wearer. The plurality of water-resistant layers of the pocket inner wall 144 can include a polyurethane laminate. For example, each water-resistant layer can be include a polyurethane laminate. FIG. 5A shows an example illustration of a laminated layer 500. Each water-resistant layer can be at least water-repellent. In some embodiments, one or more of the water-resistant layers can be water-repellent. Further, in some embodiments, one or more of the water-resistant layers can be water-proof.

The pocket outer wall 142 can be configured to retain liquid therein the pocket 140. When the undergarment 100 is worn, a portion of the pocket outer wall 142 can be at a lowest elevation of the undergarment 100. The lowest point can thus form a liquid collecting area as gravity draws liquids in the undergarment 100 downwards. The pocket outer wall 142 can be absorbent. The pocket outer wall 142 can include multiple layers. The absorbent capacity of the pocket outer wall 142 can depend on the number of layers. In some embodiments, the pocket outer wall 142 can absorb at least 75 milliliters. With the insert 180 in the main body 110, the undergarment can have an absorbent capacity of 200 milliliters.

Reference is now made to FIG. 2C, which shows a magnified, cross-sectional view 200 of the pocket 140. The pocket outer wall 142 can include at least a pocket wicking layer 202, a pocket lining layer 206, and at least one pocket absorbent layer 204 between the pocket wicking layer 202 and the pocket lining layer 206. The pocket wicking layer 202 is proximal to the pocket inner wall 144. The pocket wicking layer 202 can be formed of any appropriate fabric for drawing liquids in one direction, more specifically, in a direction toward the at least one pocket absorbent layer 204. In some embodiments, the pocket wicking layer 202 can be a knitted twill fabric. FIG. 5B shows an example illustration of a knitted twill fabric 502. In some embodiments, the pocket wicking layer 202 can be formed of polyester and spandex. For example, the pocket wicking layer 202 can be 97% polyester and 3% spandex. Other fabric contents are possible. In some embodiments, the pocket wicking layer 202 can be a polyester and spandex knitted twill fabric. The pocket wicking layer 202 can be similar to, or the same material as the insert wicking layer 402a, 402b.

In some embodiments, the at least one pocket absorbent layer 204 can include one layer. One layer can allow the pocket outer wall 142 to be less bulky and more comfortable. In some embodiments, the at least one pocket absorbent layer 204 can include two layers. In other embodiments, the at least one pocket absorbent layer 204 can include more than two layers. The at least one pocket absorbent layer 204 can be formed of any appropriate fabric for absorbing liquids. In some embodiments, the pocket absorbent layer 204 can be a microfiber loop terry fabric. FIG. 5C shows an example illustration of a microfiber loop terry fabric 504. In some embodiments, the pocket absorbent layers 204 can be formed of polyester and nylon. For example, the pocket absorbent layer 204 can be 88% polyester and 12% nylon. Other fabric contents are possible. In some embodiments, the pocket absorbent layer 204 can be a polyester and nylon microfiber loop terry fabric. The at least one pocket absorbent layer 204 can be similar to, or the same material as the plurality of insert absorbent layers 404.

The pocket lining layer 206 is at least water-resistant. The pocket lining layer 206 can be formed of any appropriate fabric to reduce liquid egress from the pocket outer wall 142, that is, out of the pocket 140. Liquid egress out of the pocket outer wall 142 would result in wetness on the wearer's clothing. In some embodiments, the pocket lining layer 206 can be formed of a plurality of water-resistant layers bonded together. The plurality of water-resistant layers of the pocket lining layer 206 can include a polyurethane laminate. FIG. 5A shows an example illustration of a laminated layer 500. Each water-resistant layer can be at least water-repellent. In some embodiments, one or more of the water-resistant layers can be water-repellent. Further, in some embodiments, one or more of the water-resistant layers can be water-proof. Other fabric contents is possible. In some embodiments, the pocket lining layer can be a knitted jersey fabric, such as a polyester knitted jersey fabric. The pocket lining layer 206 can be similar to, or the same material as the pocket inner wall 144.

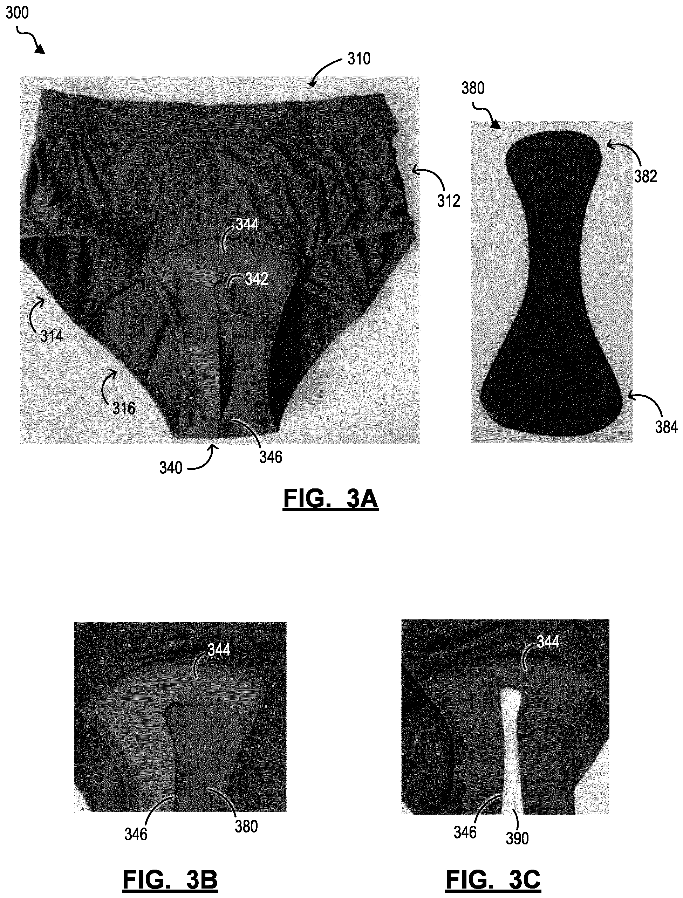

Reference is now made to FIG. 3A, which shows a front view of the interior of a main body 310 of another example undergarment 300. Similar to main body 110, main body 310 includes a front portion 312, a rear portion 314, a crotch portion 316, and a waist band 326. Similar to crotch portion 116, crotch portion 316 includes a pocket 340 and longitudinal opening 346. The pocket 340 is defined by a pocket inner wall 344 and a pocket outer wall 342. The pocket inner wall 344 has a face with a central, longitudinal opening 346 therein.

FIG. 3A further illustrates a top view of an insert 380 of the undergarment 300. Similar to insert 180, insert 380 extends from a pelvic end 382 to a buttock end 384. Also, the buttock end 384 can be larger than the pelvic end 382. Although FIG. 3B shows insert 380 being inserted in the pocket 340 of the undergarment 300, other inserts can be used with the main body 310. For example, FIG. 3C shows a disposable insert 390 positioned in the pocket 340 of the undergarment 300.

Although FIG. 1A and FIG. 3A show example undergarments 100, 300 having a main body 110, 310 and an insert 180, 380 respectively, in some embodiments, the undergarments can be a part of a kit. For example, the kit can include a plurality of inserts 180, 380. As a further example, the kit can include at least two inserts 180, 380 so that a soiled insert 180, 380 can be replaced with a clean insert 180, 380 without changing the main body 110, 310. This can be particularly desirable in institutional settings with multiple users whose undergarments can be communally washed and distributed.

In some embodiments, the main body 110, 310 can have different sizing. Further, the inserts 180, 380 can have different sizing. In some embodiments, the inserts 180, 380 can be used with multiple main body sizes. For example, in some embodiments, the main body 110, 310 can be offered in small, medium, large and extra-large sizes whereas the insert 180, 380 can be offered in a small and large size. The small size insert can be compatible with the small and medium sizes while the large size insert can be compatible with the large and extra-large sizes. Additional main body 110, 310 and insert 180, 380 are possible. This versatility with insert sizes can also be desirable in institutional settings with multiple users whose undergarments can be communally washed and distributed, or users who change sizes.

While the above description provides examples of the embodiments, it will be appreciated that some features and/or functions of the described embodiments are susceptible to modification without departing from the spirit and principles of operation of the described embodiments. Accordingly, what has been described above has been intended to be illustrative of the invention and non-limiting and it will be understood by persons skilled in the art that other variants and modifications may be made without departing from the scope of the invention as defined in the claims appended hereto. The scope of the claims should not be limited by the preferred embodiments and examples, but should be given the broadest interpretation consistent with the description as a whole.

### ITEMS

Item 1. An undergarment comprising:
a washable absorbent insert; and
a main body comprising a front portion, a rear portion opposite to the front portion, and a crotch portion having a pelvic end and a buttock end, the pelvic end of the crotch portion adjoining a bottom edge of the front portion, the buttock end of the crotch portion adjoining a bottom edge of the rear portion, the crotch portion comprising a pocket for retaining the insert therein, the pocket being defined by a pocket outer wall and a pocket inner wall, the pocket outer wall being absorbent and configured to retain liquid therein the pocket, the pocket inner wall being at least water-resistant, the pocket inner wall being proximal to a wearer when the undergarment is worn, the pocket inner wall having a face defining a longitudinal opening, the longitudinal opening being sized to allow for insertion and removal of the insert from the pocket, the longitudinal opening allowing for liquid to be deposited directly on a wicking layer therein the pocket.

Item 2. The undergarment of any preceding item, wherein the pocket outer wall forms a liquid collecting area.

Item 3. The undergarment of any preceding item, wherein:
the pocket comprises a perimeter; and
the pocket is enclosed along the perimeter.

Item 4. The undergarment of any preceding item, wherein the pocket inner wall is bonded to the pocket outer wall along the perimeter of the pocket.

Item 5. The undergarment of any preceding item, wherein the pocket inner wall comprises a plurality of water-resistant layers bonded together.

Item 6. The undergarment of any preceding item, wherein the plurality of water-resistant layers extend across the entirety of the pocket inner wall between the perimeter and the longitudinal opening.

Item 7. The undergarment of any preceding item, wherein the plurality of water-resistant layers comprise polyurethane.

Item 8. The undergarment of any preceding item, wherein each layer of the plurality of water-resistant layers is at least water-repellent.

Item 9. The undergarment of any preceding item, wherein each layer of the plurality of water-resistant layers is water-proof.

Item 10. The undergarment of any preceding item, wherein the crotch portion is seamless.

Item 11. The undergarment of any precedent item, wherein the insert comprises a plurality of insert absorbent layers sandwiched between two insert wicking layers, the insert wicking layers being configured to draw liquids toward the plurality of insert absorbent layers.

Item 12. The undergarment of any preceding item, wherein the insert wicking layer comprises a knitted twill fabric.

Item 13. The undergarment of any preceding item, wherein the knitted twill fabric comprises polyester and spandex.

Item 14. The undergarment of any preceding item, wherein each of the plurality of insert absorbent layers comprise a microfiber loop terry fabric.

Item 15. The undergarment of any preceding item, wherein the microfiber loop terry fabric comprise polyester and nylon.

Item 16. The undergarment of any preceding item, wherein the insert is reversible.

Item 17. The undergarment of any preceding item, wherein the pocket outer wall comprises at least a pocket wicking layer, a pocket lining layer, and at least one pocket absorbent layer between the pocket wicking layer and the pocket lining layer, the pocket wicking layer being configured to draw liquids toward to the at least one pocket absorbent layer, the pocket lining layer being at least water-resistant and configured to reduce liquid egress from the pocket outer wall.

Item 18. The undergarment of any preceding item, wherein the pocket wicking layer comprises a knitted twill fabric.

Item 19. The undergarment of any preceding item, wherein the knitted twill fabric comprises polyester and spandex.

Item 20. The undergarment of any preceding item, wherein each of the at least one pocket absorbent layer comprises a microfiber loop terry fabric.

Item 21. The undergarment of any preceding item, wherein the microfiber loop terry fabric comprise polyester and nylon.

Item 22. The undergarment of any preceding item, wherein the pocket lining layer comprises a plurality of water-resistant layers bonded together.

Item 23. The undergarment of any preceding item, wherein the plurality of water-resistant layers comprise polyurethane.

Item 24. The undergarment of any preceding item, wherein the pocket lining layer is at least water-repellent.

Item 25. The undergarment of any preceding item, wherein the pocket lining layer is at least water-proof.

Item 26. The undergarment of any preceding item, wherein an upper edge of the main body comprises an elastic waist band.

Item 27. The undergarment of any preceding item, wherein the front portion and the rear portion comprise a breathable fabric.

Item 28. The undergarment of any preceding item, wherein the front portion and the rear portion comprise a biodegradable fabric.

Item 29. The undergarment of any preceding item, wherein the front portion and the rear portion comprise an organic bamboo fabric.

Item 30. The undergarment of any preceding item, wherein the main body forms a pair of briefs.

Item 31. The undergarment of any preceding item, wherein the briefs are bikini briefs.

## Claims

1. An undergarment comprising:
a washable absorbent insert; and
a main body comprising a front portion, a rear portion opposite to the front portion, and a crotch portion having a pelvic end and a buttock end, the pelvic end of the crotch portion adjoining a bottom edge of the front portion, the buttock end of the crotch portion adjoining a bottom edge of the rear portion, the crotch portion comprising a pocket for retaining the insert therein, the pocket being defined by a pocket outer wall and a pocket inner wall, the pocket outer wall being absorbent and configured to retain liquid therein the pocket, the pocket inner wall being at least water-resistant, the pocket inner wall being proximal to a wearer when the undergarment is worn, the pocket inner wall having a face defining a longitudinal opening, the longitudinal opening being sized to allow for insertion and removal of the insert from the pocket, the longitudinal opening allowing for liquid to be deposited directly on a wicking layer therein the pocket.

2. The undergarment of claim 1, wherein the pocket outer wall forms a liquid collecting area.

3. The undergarment of claim 1 or 2, wherein:
the pocket comprises a perimeter; and
the pocket is enclosed along the perimeter, and wherein the pocket inner wall is bonded to the pocket outer wall along the perimeter of the pocket.

4. The undergarment of any one of claims 1 to 3, wherein the pocket inner wall comprises a plurality of water-resistant layers bonded together, and optionally wherein the plurality of water-resistant layers extend across the entirety of the pocket inner wall between the perimeter and the longitudinal opening.

5. The undergarment of claim 4, wherein the plurality of water-resistant layers comprise polyurethane.

6. The undergarment of claim 4 or 5, wherein each layer of the plurality of water-resistant layers is at least water-repellent, optionally wherein each layer of the plurality of water-resistant layers is water-proof.

7. The undergarment of any one of claims 1 to 6, wherein the crotch portion is seamless.

8. The undergarment of any one of claims 1 to 7, wherein:
the insert comprises a plurality of insert absorbent layers sandwiched between the two insert wicking layers, the insert wicking layers being configured to draw liquids toward the plurality of insert absorbent layers.

9. The undergarment of claim 8, wherein each of the insert wicking layers comprise a knitted twill fabric, optionally wherein the knitted twill fabric comprises polyester and spandex.

10. The undergarment of claim 8 or 9, wherein each of the plurality of insert absorbent layers comprise a microfiber loop terry fabric, optionally wherein the microfiber loop terry fabric comprises polyester and nylon.

11. The undergarment of any one of claims 8 to 10, wherein the insert is reversible.

12. The undergarment of any one of claims 1 to 11, wherein:
the pocket outer wall comprises at least a pocket wicking layer, a pocket lining layer, and at least one pocket absorbent layer between the pocket wicking layer and the pocket lining layer, the pocket wicking layer configured to draw liquids toward to the at least one pocket absorbent layer, the pocket lining layer being at least water-resistant and configured to reduce liquid egress from the pocket outer wall.

13. The undergarment of claim 12, wherein the pocket wicking layer comprises a knitted twill fabric, optionally wherein the knitted twill fabric comprises polyester and spandex, and wherein each of the at least one pocket absorbent layers comprise a microfiber loop terry fabric, optionally wherein the microfiber loop terry fabric comprises polyester and nylon.

14. The undergarment of claim 12 or 13, wherein the pocket lining layer comprises a plurality of water-resistant layers bonded together, optionally wherein the plurality of water-resistant layers comprise polyurethane.

15. The undergarment of any one of claims 1 to 14, wherein the front portion and the rear portion comprise at least one of a breathable and a biodegradable fabric, optionally wherein the front portion and the rear portion comprise an organic bamboo fabric.
